# EUROPEAN PATENT SPECIFICATION

(11) **EP 3 366 272 B1**
(45) Date of publication and mention of the grant of the patent: **30.10.2019**
(21) Application number: 18155397.5
(22) Date of filing: 06.02.2018
(51) Int. Cl.: A61K 8/25, A61K 8/39, A61Q 11/00, A61K 8/86

(54) **ORAL CARE COMPOSITIONS**
MUNDPFLEGEZUSAMMENSETZUNGEN
COMPOSITIONS DE PROTECTION ORALE

(30) Priority: 28.02.2017 EP 17158274
(43) Date of publication of application: 29.08.2018
(73) Proprietor: Unilever PLC, London, Greater London EC4Y 0DY (GB); Unilever N.V., 3013 AL Rotterdam (NL)
(72) Inventor: BRANNON, Helen, Louise, Bebington, Wirral, Merseyside CH63 3JW (GB); MARRIOTT, Robert, Edward, Bebington, Wirral, Merseyside CH63 3JW (GB); WILSON, William, John, Bebington, Wirral, Merseyside CH63 3JW (GB)
(74) Representative: Tansley, Sally Elizabeth

(56) References cited:
- WO-A1-2009/032406
- WO-A1-2011/068513
- WO-A1-2015/036533
- JP-A- 2009 114 125
- US-A1- 2009 238 769
- DATABASE GNPD [Online] MINTEL; March 2016 (2016-03), "Zero Fluoride Toothpaste", XP002768974, Database accession no. 3702789
- SHUBIN AD, FELONG TJ, GRAUNKE D, OVITT C,BENOIT DS: "Development of poly(ethylene glycol) hydrogels for salivary gland tissue engineering applications.", TISSUE ENG PART A., vol. 21, no. 11-12, June 2015 (2015-06), - June 2015 (2015-06), pages 1733-1751, XP002768975,
- AKIKO MORITO ET AL: "Protective effects of polysaccharides and polyhydric alcohols in a dry mouth model in cultured cells", SUPPORTIVE CARE IN CANCER, SPRINGER-VERLAG, DE, vol. 20, no. 4, 9 April 2011 (2011-04-09), pages 725-731, XP035026633, ISSN: 1433-7339, DOI: 10.1007/S00520-011-1135-7

## Description

### Field of the Invention

The present invention is concerned with oral care compositions. More particularly, the present invention is concerned with oral care compositions that improve the lubricity of the mouth.

### Background of the Invention

The dental pellicle is a protein film that forms on the surface of teeth. It helps protect the teeth from external attacks, such as acid erosion and wear.

The dental pellicle can be removed when the teeth are brushed. The present application relates to compositions that clean the teeth and help maintain the dental pellicle and hence the lubricity of the mouth.

### Description of the Invention

The present application relates to the non-therapeutic use of a non-ionic surfactant comprising polyethylene glycol ether of a fatty alcohol, in a composition to promote lubricity of the mouth.

### Detailed Description of the Invention

The present invention relates to a composition that deposits on the existing pellicle layer and thus helps protect the pellicle. It is thought that this deposition results in a change in the surface of the pellicle and helps build up an increased pellicle layer or enhance the layer. The enhanced layer results in an increased lubrication which is, in the context of the present invention, seen as an enhancement.

The present invention relates to a method of maintaining/enhancing/lubricating the salivary pellicle, preferably the salivary pellicle of the teeth.

The composition of the invention comprises a non-ionic surfactant.comprising polyethylene glycol ethers of fatty alcohols in particular polyethylene glycol ethers having from 20 to 40 ethylene oxide groups per unit. An example of such a suitable surfactant includes the group of surfactants known as Steareth surfactants such as Steareth 30. Preferably the level of non-ionic surfactant is from 0.2 to 7 wt% of the total composition, more preferably from 1 to 5 wt% of the total composition.

Although other surfactants may be present it is preferred if they are limited to levels below 0.5 wt% of the total composition preferably less than 0.1 wt% of the total composition. It is particularly preferred if the composition is substantially free of anionic surfactants that it comprises less than 0.05 wt% of anionic surfactant.

The oral care composition, especially also comprises natural gums (such as carrageenan, gum karaya, guar gum, xanthan gum, gum arabic, and gum tragacanth). Carrageenan is particularly preferred.

A preferred carrageenan gum having has major amounts of kappa and lambda constituents. Carrageenan is a high molecular weight linear polysaccharide derived from sea plants which makes up approximately 2-7% of the plant and is found between the cellulosic fibres and is composed of repeating galactose units, and 3,6 anhydrogalactose (3,6-AG), both sulfated and nonsulfated, joined by alternating 1-3, β 1-4 glycosidic linkages. A preferred carrageenan gum is available commercially from FMC Corporation under the Trademark Viscarin TP-206 which typically contains 62% lambda, 30% kappa and 8% iota forms.

Preferably the level of carrageenan is from 0.1 wt% to 1.5 wt% of the total composition, more preferably from 0.3 wt% to 1.0 wt%.

Compositions of the invention may also comprise gums such as xanthan gum.

The total natural gums are present at levels from 0.1 wt% to 4 wt%, more preferably from 0.1 to 3 wt% of the total composition.

Additionally, other structurant agents such as binders and thickening agents may be present. These structurants will generally be present in an amount of from 0.1 to 1% by weight based on the total weight of the dentifrice. Suitable binders or thickening agents include carboxyvinyl polymers (such as polyacrylic acids cross-linked with polyallyl sucrose or polyallyl pentaerythritol), hydroxyethyl cellulose, hydroxypropyl cellulose, water soluble salts of cellulose ethers (such as sodium carboxymethyl cellulose and sodium carboxymethyl hydroxyethyl cellulose).

Compositions of the invention may also comprise other proteins and enzymes. Preferably these proteins and or enzymes are present at total levels from 0.001 to 0.5 wt% of the total composition, preferably from 0.005 to 0.1 wt% of the total composition.

Examples of suitable product forms for compositions of the invention include dentifrices, mouthwashes, mouthsprays and liquid formulations.

Preferred product forms for compositions of the invention are those which are suitable for brushing and/or rinsing the surfaces of the oral cavity. Dentifrices/toothpastes are preferred.

In such preferred product forms, the amount of water and/or polyhydric alcohol will generally be at least 10 percent, preferably at least 30 percent, more preferably at least 50 percent by total weight water and/or polyhydric alcohol based on the total weight of the composition. Preferably the level of water and/or polyhydric alcohol will be less than 90 wt% of the total composition, more preferably less than 85 wt%.

An example of a preferred type of product form in the context of the present invention is a dentifrice. The term "dentifrice" generally denotes formulations which are used to clean the surfaces of the oral cavity. The dentifrice is an oral composition that is not intentionally swallowed for purposes of systemic administration of therapeutic agents, but is applied to the oral cavity, used to treat the oral cavity and then expectorated. Typically, the dentifrice is used in conjunction with a cleaning implement such as a toothbrush, usually by applying it to the bristles of the toothbrush and then brushing the accessible surfaces of the oral cavity. Preferably the dentifrice is in the form of a paste or a gel (or a combination thereof).

A dentifrice composition according to the invention will usually contain, as the aqueous continuous phase, a mixture of water and polyhydric alcohol in various relative amounts, with the amount of water generally ranging from 10 to 50% by weight (based on the total weight of the dentifrice) and the amount of polyhydric alcohol generally ranging from 20 to 60% by weight (based on the total weight of the dentifrice).

Typical polyhydric alcohols for use in the oral care composition, particularly a dentifrice composition according to the invention include humectants such as glycerol, sorbitol, polyethylene glycol, polypropylene glycol, propylene glycol, xylitol (and other edible polyhydric alcohols), hydrogenated partially hydrolyzed polysaccharides and mixtures thereof. Glycerol and sorbitol are preferred, sorbitol being particularly preferred.

The oral care composition, in particular dentifrice compositions comprises abrasive materials. Abrasive materials will generally be present in an amount of from 0.5 to 75%, preferably 3 to 60 % by weight based on the total weight of the dentifrice. Suitable abrasive cleaning agents include silica xerogels, hydrogels and aerogels and precipitated particulate silicas; calcium carbonate, dicalcium phosphate, tricalcium phosphate, calcined alumina, sodium and potassium metaphosphate, sodium and potassium pyrophosphates, sodium trimetaphosphate, sodium hexametaphosphate, particulate hydroxyapatite and mixtures thereof. Silicas are particularly preferred.

Compositions of the invention may also comprise thickeners such as finely divided silicas, hectorites, calcium carbonates, colloidal magnesium aluminium silicates and mixtures thereof.

Compositions of the present invention may also contain further optional ingredients customary in the art such as fluoride ion sources, anticalculus agents, buffers, flavouring agents, bleaching agents, sweetening agents, colouring agents, opacifying agents, preservatives, antisensitivity agents and antimicrobial agents.

Compositions of the invention may comprise a zinc ion. Preferably the sources of zinc ions are zinc chloride, zinc acetate, zinc gluconate, zinc sulphate, zinc fluoride, zinc citrate, zinc lactate, zinc oxide, zinc monoglycerolate, zinc tartrate, zinc pyrophosphate zinc maleate and mixtures thereof.

To clean the surfaces of the oral cavity, the composition according to the invention is topically applied to the oral cavity surfaces to be cleaned.

Preferably the composition according to the invention is topically applied to the oral cavity surfaces to be cleaned and then agitated by brushing and/or rinsing.

The invention will now be illustrated by the following non-limiting Examples: Examples of the invention are illustrated by a number, comparative Examples are illustrated by a letter.

### Examples

Examples were prepared as follows:

**Table 1**

| **Material** | **Amount % W/W** | | |
|---|---|---|---|
| | **Example 1** | **Example A** | **Example B** |
| Sorbitol | 28.50 | 28.50 | 28.50 |
| Sodium Fluoride | 0.32 | 0.32 | 0.32 |
| Citric Acid | 0.25 | 0.25 | 0.25 |
| Disodium Phosphate | 0.50 | 0.50 | 0.50 |
| Titanium Dioxide | 0.50 | 0.50 | 0.50 |
| Thickening silica (synthetic amorphous silica) | 4.80 | 4.80 | 4.80 |
| Glycerol/Glycerine | 5.00 | 5.00 | 5.00 |
| Xanthan | 1.00 | 1.00 | 1.00 |
| Carrageenan | 0.70 | 0.70 | 0.70 |
| Stearate 30 | 3.00 | 0.00 | 0.00 |
| Flavour | 0.80 | 0.80 | 0.80 |
| Sodium Lauryl Sulphate | 0.00 | 0.00 | 1.80 |
| Abrasive silica (synthetic amorphous silica) | 16.00 | 16.00 | 16.00 |
| Water and minors | To 100 | To 100 | To 100 |

### Eldredge Tribometer Setup:

Load: 20g
Speed: 1.8 mm/s
Base substrate: HAP Disk - 0.5" diameter, 0.08" thick (Clarkson Chromatography)
Probe: Synthetic Sapphire

### Irradiation of Saliva

Pooled saliva samples were gamma- irradiated on a Cobalt 60 turntable by Synergy Health at Moray Road, Elgin Industrial Estate, Swindon SN2 8XS. The gamma sterilization process uses Cobalt 60 radiation at a dose of 40kGy, to kill microorganisms on a variety of different products.

### Pellicle Formation

Hydroxy Apatite (HAP) disks [0.5 inch diameter, 0.08 inch thick] were submersed in ethanol for 20 mins before being removed and placed in an oven to dry at 40°C overnight. Disks were removed, and placed into a 24-well plate. Irradiated saliva was added to the well plate to fully cover the HAP disk and the well plate placed in the incubator at 37 °C shaking slowly for 2 hours. The disks were removed.

### Paste Treatment

The Example pastes were mixed with water in a 0.3 ratio using a FastPrep (MP Biomedicals) at 8000 rpm for 2 x 30s. The resulting slurries were placed into a centrifuge at 10,500 rpm for 10 mins. The resulting supernatant solutions were immediately pipetted into new clean sterilised vials. The supernatant solutions were pipetted into a 24-well plate and the HAP disks (with pellicle formed) were placed into the wells submersed in solution for 1 min (whilst being lightly agitated) before measuring the friction. When rinsing of the HAP disks was required after treatment, they were submersed in water for 1 minute (whilst being lightly agitated) before measuring the friction.

### Protocol

The following experimental protocol was followed. Note different HAP disks were measured for each stage because the Eldredge tribometer is a contact measurement and can potentially cause surface damage, therefore only one friction measurement can be done per HAP disk:
Step 1: HAP with no pellicle
Step 2: form pellicle
Step 3: rinse (agitated gently in agitator for 1 min)
Step 4: Place HAP disk in supernatant solution for 1 min
Step 5: rinse (agitated gently in agitator for 1 min)
Step 6: Re-make pellicle (place in saliva again in incubator for 2 hrs)
Step 7: Rinse (agitated gently in agitator for 1 min)

Table 2 shows the normalised friction measurements of HAP disks treated according to steps 6 or step 7 compared with step 5.

**Table 2**

| | **Experiment** | |
|---|---|---|
| | **Friction with no rinse (step 6)** | **Friction with rinse (step 7)** |
| **Example B** | 56.72878637 | 65.39265011 |
| **Example 1** | -17.08168039 | -16.22093399 |
| **Example A** | -11.44904237 | -6.139392439 |
| | | |

| | **Standard Error** | |
|---|---|---|
| | **Step 6** | **Step 7** |
| **Example B** | 6.593322828 | 4.086356714 |
| **Example 1** | 4.970040428 | 7.439674378 |
| **Example A** | 6.314940089 | 3.743507782 |

The results of table 2 demonstrate that Example 1 comprising Steareth-30 based paste preserves the low friction and hence the lubricity, comparable to that of the paste containing no surfactant Example A. However, the SLS based paste - Example B significantly increases the friction compared to Example 1 and Example A.

## Claims

1. Non-therapeutic use of a non-ionic surfactant comprising polyethylene glycol ether of a fatty alcohol, in a composition to promote lubricity of the mouth.

2. Non-therapeutic use according to any preceding claim in which the non-ionic surfactant is Steareth 30.

3. Non-therapeutic use according to any preceding claim in which the composition further comprises a polyhydric alcohol.

4. Non-therapeutic use according to any preceding claim in which the composition comprises less than 0.05 wt% of anionic surfactant.

5. Non-therapeutic use according to any preceding claim in which the non-ionic surfactant is formulated as a dentifrice composition.

6. Non-therapeutic use according to any preceding claim in which the composition further comprises an abrasive silica.

7. Non-therapeutic use according to any preceding claim in which the non-ionic surfactant further enhances the lubricated feel of the mouth.

## Patentansprüche

1. Nicht-therapeutische Verwendung eines nicht-ionischen Tensids, umfassend Polyethylenglykolether eines Fettalkohols in einer Zusammensetzung, um die Gleitfähigkeit im Mund zu fördern.

2. Nicht-therapeutische Verwendung nach einem vorhergehenden Anspruch, in welchem das nicht-ionische Tensid Steareth 30 darstellt.

3. Nicht-therapeutische Verwendung nach irgendeinem vorhergehenden Anspruch, bei welcher die Zusammensetzung ferner einen mehrwertigen Alkohol umfasst.

4. Nicht-therapeutische Verwendung nach irgendeinem vorhergehenden Anspruch, in welcher die Zusammensetzung weniger als 0,05 Gew.-% anionisches Tensid umfasst.

5. Nicht-therapeutische Verwendung nach irgendeinem vorhergehenden Anspruch, in welcher das nicht-ionische Tensid als Zahnputzmittelzusammensetzung formuliert ist.

6. Nicht-therapeutische Verwendung nach irgendeinem vorhergehenden Anspruch, in welcher die Zusammensetzung ferner ein abrasives Siliziumdioxid umfasst.

7. Nicht-therapeutische Verwendung nach irgendeinem vorhergehenden Anspruch, in welcher das nicht-ionische Tensid ferner das Mundgefühl der Gleitfähigkeit verbessert.

## Revendications

1. Utilisation non thérapeutique d'un tensioactif non-ionique comprenant un éther de polyéthylèneglycol et d'un alcool gras, dans une composition pour promouvoir le pouvoir lubrifiant de la bouche.

2. Utilisation non thérapeutique selon la revendication précédente, dans laquelle le tensioactif non-ionique est le Steareth 30.

3. Utilisation non thérapeutique selon l'une quelconque des revendications précédentes, dans laquelle la composition comprend en outre un alcool polyvalent.

4. Utilisation non thérapeutique selon l'une quelconque des revendications précédentes, dans laquelle la composition comprend moins de 0,05 % en poids de tensioactif anionique.

5. Utilisation non thérapeutique selon l'une quelconque des revendications précédentes, dans laquelle le tensioactif non-ionique est formulé sous la forme d'une composition de dentifrice.

6. Utilisation non thérapeutique selon l'une quelconque des revendications précédentes, dans laquelle la composition comprend en outre une silice abrasive.

7. Utilisation non thérapeutique selon l'une quelconque des revendications précédentes, dans laquelle le tensioactif non-ionique amplifie en outre la sensation de lubrification de la bouche.
